# EUROPÄISCHE PATENTANMELDUNG

(11) **EP 3 922 299 A1**
(43) Veröffentlichungstag der Anmeldung: **15.12.2021**
(21) Anmeldenummer: 20179453.4
(22) Anmeldetag: 11.06.2020
(51) Int. Cl.: A61M 60/178, A61M 60/216, A61M 60/878

(54) **VERBINDUNGSSYSTEM ZUR ENERGIE- UND/ODER DATENÜBERTRAGUNG VON UND/ODER ZU EINER IMPLANTIERBAREN BLUTPUMPE UND HERZUNTERSTÜTZUNGSSYSTEM**

(71) Anmelder: Berlin Heart GmbH, 12247 Berlin (DE)
(72) Erfinder: Austilat, Konstantin, 14050 Berlin (DE); Robbenmenke, Christian, 10965 Berlin (DE)
(74) Vertreter: Pfenning, Meinig & Partner mbB

(57) **Zusammenfassung**

Die Anmeldung betrifft ein Verbindungssystem (12) zur Energie- und/oder Datenübertragung von und/oder zu einer implantierbaren Blutpumpe (2). Das vorgeschlagene Verbindungssystem (12) umfasst eine erste Verbindungseinheit (14), die mit der Blutpumpe (2) verbindbar ist, und eine zweite Verbindungseinheit (16), die mit einer extrakorporalen Steuer- und/oder Energieeinheit (5) verbindbar ist. Die erste Verbindungseinheit (14) kann insbesondere durch eine implantierbare Leitung (13) mit der Blutpumpe (2) verbindbar sein. Die zweite Verbindungseinheit (16) kann durch eine transkutane Leitung (6) mit der extrakorporalen Steuer- und/oder Energieeinheit (5) verbindbar sein. Die erste Verbindungseinheit (14) und die zweite Verbindungseinheit (16) sind zur drahtlosen Energie- bzw. Datenübertragung drahtlos miteinander koppelbar. Außerdem sind die erste Verbindungseinheit (14) und die zweite Verbindungseinheit (16) implantierbar ausgeführt sind, sodass sowohl die erste Verbindungseinheit (14) als auch die zweite Verbindungseinheit (16) zur Verwendung innerhalb eines Körpers eines Patienten eingerichtet ist.

## Beschreibung

Die vorliegende Anmeldung liegt auf dem Gebiet der Medizintechnik und insbesondere auf dem Gebiet implantierbarer Blutpumpen zur Unterstützung einer Herzfunktion. Die Anmeldung betrifft ein Verbindungssystem zur Energie- und/oder Datenübertragung von und/oder zu einer implantierbaren Blutpumpe und ein Herzunterstützungssystem, das ein solches Verbindungssystem aufweist.

Herzunterstützungssysteme mit implantierbaren Blutpumpen sind aus dem Stand der Technik bekannt. Diese Herzunterstützungssysteme können zum Einsatz kommen, wenn eine Herzfunktion eines Patienten unterstützt oder ersetzt werden muss. Gängige Systeme, die hierbei verwendet werden, sind sogenannte VAD (ventricular assist devices). Derartige Systeme können beispielsweise als sog. LVAD (left ventricular assist device), RVAD (right ventricular assist device) oder BiVAD (bi-ventricular assist device) ausgeführt sein. Diese Systeme umfassen neben der implantierbaren Blutpumpe, die im Betrieb in dem Patienten implantiert ist, in der Regel eine beispielsweise außerhalb eines Körpers des Patienten angeordnete und mit der Blutpumpe über eine transkutane Leitung (Driveline) verbundene Steuereinheit. Über die transkutane Leitung können Daten zwischen der Blutpumpe und der Steuereinheit ausgetauscht werden. Zudem ist in der Regel eine außerhalb des Körpers angeordnete Energieeinheit vorgesehen, die ebenfalls über die transkutane Leitung mit der Blutpumpe verbunden ist und über welche die Blutpumpe mit elektrischer Energie versorgt wird. Die Blutpumpe umfasst in der Regel einen Motor mit einem Stator und einem mit einer Beschaufelung versehenen Rotor, der in einem Strömungskanal der Blutpumpe angeordnet ist. Durch von dem Steuergerät gelieferte Energie kann der Motor der Blutpumpe angetrieben werden, indem beispielsweise ein Stromfluss in Wicklungen des Stators erzeugt wird, durch den der Rotor mitsamt Beschaufelung zum Fördern von Blut des Patienten in Drehung versetzt wird.

Da die transkutane Leitung, die in der Regel als teilweise implantierbares Kabel ausgeführt ist, durch die Haut des Patienten geleitet wird, ist eine Durchtrittsstelle vonnöten. Die Durchtrittsstelle ist nachteiligerweise anfällig für Infektionen. Um Durchtrittsstellen zu vermeiden, ist es denkbar, Verbindungssysteme vorzusehen, durch die eine berührungslose Energie- oder Datenübertragung durch die Haut hindurch möglich ist. Solche sogenannten TET-Systeme (Transcutaneous Energy Transfer) sehen in der Regel eine implantierte Spule vor, die mit der Blutpumpe über ein Kabel verbunden ist. Außerdem ist eine extrakorporale Spule vorgesehen, die induktiv mit der implantierten Spule gekoppelt ist. Diese extrakorporale Spule ist außerhalb des Körpers angeordnet und über eine vollständig außerhalb des Patientenkörpers angeordnete Leitung mit der Energie- und der Steuereinheit verbunden. Für eine solche Art der transkutanen Energieübertragung ergeben sich jedoch geringe Wirkungsgrade, die zu einer hohen Erwärmung des umliegenden Gewebes führen. Außerdem müssen die implantierte Spule und die extrakorporale Spule notwendigerweise sehr genau zueinander platziert werden, um eine zuverlässige Übertragung zu gewährleisten. Derartige Systeme benötigen zur Gewährleistung einer Ausfallsicherheit zusätzlich eine implantierbare Batterie, was zu einer vergleichsweise großen Komplexität derartiger Systeme beiträgt.

Auch weitere Ansätze sind denkbar, durch welche Komplikationen im Zusammenhang mit den oben beschriebenen Infektionen der Durchtrittsstelle gelindert werden. Beispielsweise ist es denkbar, die Driveline, die die extrakorporalen Steuer- und Energieeinheiten mit der implantierten Blutpumpe verbindet, mit einem implantierten Steckverbinder auszustatten. Dies hätte den Vorteil, dass im Falle einer Infektion der Durchtrittsstelle nur ein kurzer Kabelabschnitt zwischen der Energie- bzw. Steuereinheit und dem implantierbaren Steckverbinder getauscht werden müsste und die Blutpumpe hierbei im Patienten verbleiben könnte. Es wäre somit nur ein verhältnismäßig kleiner operativer Eingriff nötig. Zusätzlich kann bei dieser Lösung auf die implantierte Batterie mit den damit verbundenen Nachteilen verzichtet werden. Implantierbare Steckverbinder sind technisch jedoch vergleichsweise schwierig zu realisieren. Hierbei sind vor allem die Dichtigkeit, Korrosionsanfälligkeit sowie die Isolation der Leiter technische Probleme, die Herausforderungen darstellen.

Vor dem Hintergrund der oben beschriebenen Aspekte ist es eine Aufgabe der vorliegenden Anmeldung ein verbessertes Verbindungssystem zur Energie- und/oder Datenübertragung von und/oder zu einer implantierbaren Blutpumpe sowie ein entsprechend verbessertes Herzunterstützungssystem vorzuschlagen. Insbesondere ist es eine Aufgabe der vorliegenden Anmeldung ein Verbindungssystem vorzuschlagen, das ein vergleichsweise einfach aufgebautes Herzunterstützungssystem ermöglicht, bei dem im Zusammenhang mit Infektionen der Hautdurchtrittsstelle auftretende Probleme gelindert werden. Zudem soll das Risiko von Gewebeschädigungen im Vergleich zum oben beschriebenen Stand der Technik gering gehalten werden.

Diese Aufgaben werden gelöst durch ein Verbindungssystem mit den Merkmalen des unabhängigen Anspruchs 1 und durch ein Herzunterstützungssystem mit den Merkmalen eines weiteren Anspruchs. Vorteilhafte Weiterbildungen ergeben sich mit den Merkmalen der abhängigen Ansprüche und der Ausführungsbeispiele.

Das vorgeschlagene Verbindungssystem zur Energie- und/oder Datenübertragung von und/oder zu einer implantierbaren Blutpumpe umfasst eine erste Verbindungseinheit, die mit der Blutpumpe verbindbar ist, und eine zweite Verbindungseinheit, die mit einer extrakorporalen Steuer- und/oder Energieeinheit verbindbar ist. Die erste Verbindungseinheit kann insbesondere durch eine implantierbare Leitung mit der Blutpumpe verbindbar sein. Die zweite Verbindungseinheit kann durch eine transkutane Leitung mit der extrakorporalen Steuer- und/oder Energieeinheit verbindbar sein. Die transkutane Leitung kann vollständig oder zumindest in einem zu implantierenden Abschnitt implantierbar ausgeführt sein und insbesondere an seiner Außenseite biokompatibles Material aufweisen oder aus biokompatiblem Material bestehen. Die erste Verbindungseinheit und die zweite Verbindungseinheit sind zur drahtlosen Energie- bzw. Datenübertragung drahtlos miteinander koppelbar. Außerdem sind die erste Verbindungseinheit und die zweite Verbindungseinheit implantierbar ausgeführt sind, sodass sowohl die erste Verbindungseinheit als auch die zweite Verbindungseinheit zur Verwendung innerhalb eines Körpers eines Patienten eingerichtet ist.

Die vorliegende Anmeldung betrifft auch ein Herzunterstützungssystem, das ein wie oben oder unten beschriebenes Verbindungssystem umfasst. Weiterhin kann das Herzunterstützungssystem die Blutpumpe und/oder die extrakorporale Steuer- und/oder Energieeinheit umfassen. Die erste Verbindungseinheit kann mit der implantierbaren bzw. implantierten Leitung mit der Blutpumpe verbindbar sein. Die zweite Verbindungseinheit kann über die vollständig oder zumindest teilweise implantierbare transkutane Leitung, insbesondere lösbar, mit der extrakorporalen Steuer- und/oder Energieeinheit verbindbar sein. Die transkutane Leitung tritt bei einer Verwendung des Herzunterstützungssystems in der Regel durch eine Durchtrittsstelle in der Haut.

Das vorgeschlagene Verbindungssystem ermöglicht den einfachen Austausch von lediglich der transkutanen Leitung und der zweiten Verbindungseinheit, wenn eine Infektion der Durchtrittsstelle auftritt. Gegenüber implantierbaren Steckverbindern weist das vorgeschlagene Verbindungssystem der Vorteil auf, dass die Verbindungseinheiten vollständig gekapselt ausgeführt sein können. Somit werden bei Steckverbindern im Zusammenhang mit einer Verschmutzung und Korrosion der elektrischen Steckkontakte auftretende Probleme sowie generell im Zusammenhang mit durch eintretende Flüssigkeit hervorgerufene Probleme wie beispielsweise Kurzschlüsse durch das vorgeschlagene Verbindungssystem überwunden. Insbesondere kann es vorgesehen sein, dass das Verbindungssystem zur Kopplung der Verbindungseinheiten miteinander, wenn die transkutane Leitung und die vollständig implantierte Leitung zur Verbindung der Blutpumpe mit dem Verbindungssystem verbunden sind, keine außenliegenden, insbesondere keine aktiven, elektrischen Leiter, insbesondere Steckkontakte, aufweist. Zudem werden durch das vorgeschlagene Verbindungssystem Probleme, die bei anderen Systemen im Zusammenhang mit der Abdichtung und Isolation außenliegender Steckkontakte auftreten können, vermieden.

Im Vergleich zu transkutanen Energieübertragungssystemen (TET) ermöglicht das vorgeschlagene Verbindungssystem deutlich erhöhte Wirkungsgrade. Dies wird dadurch erreicht, dass die Verbindungseinheiten bei ihrer Verwendung sehr nah, insbesondere berührend, angeordnet sind. Somit werden Probleme im Zusammenhang mit einem durch den Abstand beispielsweise einer Sender- und einer Empfängerspule zueinander verminderter Kopplungsfaktoren und verminderter Wirkungsgrade vermieden. Insbesondere wird durch das vorgeschlagene Verbindungssystem vermieden, dass aufgrund reduzierter Wirkungsgrade eine thermische Gewebebelastung und/oder Gewebeschädigung auftritt. Das vorgeschlagene Verbindungssystem ermöglicht zudem, dass das Herzunterstützungssystem nicht notwendigerweise eine implantierte Batterie für eine Notversorgung aufweist.

Bei einem Verfahren, das ebenfalls Gegenstand der vorliegenden Anmeldung sein kann, wird zunächst ein wie oben oder unten beschriebenes Herzunterstützungssystem bereitgestellt. Insbesondere nachdem eine Infektion der Durchtrittsstelle festgestellt wurde, können, gegebenenfalls nach einem Lösen der ersten Verbindungseinheit von der zweiten Verbindungseinheit, die zweite Verbindungseinheit und die transkutane Leitung entfernt und gegen eine weitere zweite Verbindungseinheit und eine weitere transkutane Leitung ausgetauscht werden. Hierbei kann es vorgesehen sein, dass die transkutane Leitung von der extrakorporalen Steuer- und/oder Energieeinheit gelöst und dass die weitere transkutane Leitung mit der extrakorporalen Steuer- und/oder Energieeinheit verbunden wird.

Es kann vorgesehen sein, dass die erste und zweite Verbindungseinheit induktiv und/oder kapazitiv drahtlos miteinander koppelbar sind. Beispielsweise kann die erste Verbindungseinheit zumindest eine Spule aufweisen. Zudem kann die zweite Verbindungseinheit zumindest eine Spule aufweisen. Die erste Verbindungseinheit und die zweite Verbindungseinheit können über die Spulen zur drahtlosen Energie- bzw. Datenübertragung insbesondere induktiv miteinander koppelbar sein.

Den Begriff implantierbar sowie das Eingerichtetsein zur Verwendung innerhalb des Körpers des Patienten versteht der Fachmann im Zusammenhang mit der vorliegenden Anmeldung strukturell einschränkend. Beispielsweise weiß der Fachmann für Medizintechnik, welche Anforderungen an implantierbare Geräte in struktureller Hinsicht zu stellen sind.

Die erste Verbindungseinheit und die zweite Verbindungseinheit sind in der Regel in separaten Gehäusen aufgenommen. Es kann vorgesehen sein, dass die erste Verbindungseinheit ein Gehäuse aufweist und dass die zweite Verbindungseinheit ein Gehäuse aufweist, wobei das Gehäuse der ersten Verbindungseinheit und/oder das Gehäuse der zweiten Verbindungseinheit flüssigkeitsdicht, insbesondere dicht für Körperflüssigkeiten, beispielsweise blutdicht, ausgeführt sind. In einigen Ausführungen sind die Gehäuse baugleich ausgeführt. Da die Verbindungseinheiten für eine Implantation vorgesehen sind, sind diese platzsparend ausgeführt. Insbesondere kann es vorgesehen sein, dass die Verbindungseinheiten im Wesentlichen hohlraumfrei sind und/oder dass die Gehäuse keine Lufträume einschließen. In dem Gehäuse der ersten Verbindungseinheit ist beispielsweise die Spule der ersten Verbindungseinheit aufgenommen, und in dem Gehäuse der zweiten Verbindungseinheit ist beispielsweise die Spule der zweiten Verbindungseinheit aufgenommen. Das Gehäuse der ersten Verbindungseinheit und/oder das Gehäuse der zweite Verbindungseinheit ist typischerweise biokompatibel. Insbesondere kann das Gehäuse der ersten Verbindungseinheit und/oder das Gehäuse der zweiten Verbindungseinheit eine vollständig biokompatible äußere Oberfläche aufweisen. Der Anschluss der transkutanen Leitung an die zweite Verbindungseinheit und/oder der Anschluss der implantierbaren Leitung zur Verbindung der ersten Verbindungseinheit mit der Blutpumpe ist in der Regel ebenfalls flüssigkeitsdicht und/oder implantierbar ausgeführt.

Es kann vorgesehen sein, dass das Verbindungssystem ein mechanisches Verbindungsmittel aufweist, durch das die erste Verbindungseinheit und die zweite Verbindungseinheit lösbar miteinander verbindbar, insbesondere nicht verschiebbar und/oder starr aneinander befestigbar, sind. Durch das mechanische Verbindungsmittel kann eine mechanische Fixierung der Verbindungseinheiten erreicht werden, sodass die Verbindungseinheiten zur zuverlässigen Energie- bzw. Datenübertragung in Position gehalten werden und sodass ein ungewolltes Ablösen im Patientenkörper verhindert wird. Zur Verhinderung des ungewollten Ablösens kann es vorgesehen sein, dass das mechanische Verbindungsmittel eine Verbindung zwischen der ersten Verbindungseinheit und der zweiten Verbindungseinheit vermittelt, deren Lösen eine Kraftwirkung von zumindest 10 N, insbesondere zumindest 15 N, erfordert. Es kann vorgesehen sein, dass das mechanische Verbindungsmittel ein Schraubverschluss, ein Bajonettverschluss, ein Rastverschluss oder und/oder ein Schnappverschluss ist.

Es kann auch vorgesehen sein, dass das mechanische Verbindungsmittel ein äußeres Gehäuse ist, das eingerichtet ist, die erste Verbindungseinheit und die zweite Verbindungseinheit aufzunehmen. Das äußere Gehäuse kann beispielsweise nach einer Ausrichtung der Verbindungseinheiten zueinander um beide Verbindungseinheiten gelegt werden. Das hat den Vorteil, dass bei einem Tausch einer Seite, d.h. einer Verbindungseinheit, beide Seiten mit dem Lösen des äußeren Gehäuses frei zugänglich sind und der Tausch somit einfacher ist. Umfasst das äußere Gehäuse für beide Spulen z.B. Silikon oder eine Silikonbeschichtung, wird zusätzlich ein Einwachsen vermieden, was ebenfalls einen einfacheren Tausch ermöglicht.

Es kann vorgesehen sein, dass das Verbindungssystem ein mechanisches Ausrichtungsmittel aufweist. Durch das mechanische Ausrichtungsmittel können die erste Verbindungseinheit und die zweite Verbindungseinheit zueinander ausrichtbar sein, sodass die erste Verbindungseinheit und die zweite Verbindungseinheit in einer vordefinierten Ausrichtung, insbesondere Position und Orientierung, zueinander zu liegen kommen. Das Ausrichtungsmittel kann beispielsweise gewährleisten, dass die erste Verbindungseinheit und die zweite Verbindungseinheit so ausgerichtet werden, dass die gegebenenfalls vorgesehenen Spulen in einem gewünschten Ausrichtung zueinander zu liegen kommen, wenn die erste Verbindungseinheit relativ zur zweiten Verbindungseinheit angeordnet wird. Somit können ungewünschte laterale Abweichungen in der Positionierung der Verbindungseinheiten zueinander, wie sie beispielsweise bei TET-Systemen auftreten können, verhindert werden.

Das Verbindungssystem wird hierbei typischerweise, insbesondere vollständig, in einer Hauttasche und/oder in der Subkutis des Patienten angeordnet. Es kann aber auch vorgesehen sein, dass das Verbindungssystem, insbesondere vollständig, unter der oberflächlichen Faszie platziert wird. Auf diese Weise kann sich das Verbindungssystem die Wirkung der Faszie als zusätzliche, natürliche Infektionsbarriere zunutze machen. Das Verbindungsystem kann insbesondere in seinen räumlich-körperlichen Eigenschaften derart ausgebildet sein, dass es eingerichtet und geeignet ist, insbesondere vollständig in der Subkutis oder unterhalb der oberflächlichen Faszie eines Patienten angeordnet zu werden. Die transkutane Leitung kann zu diesem Zweck eine entsprechend geeignete Mindestlänge aufweisen. Die transkutane Leitung ist jedoch typischerweise nicht länger als 1 m. Um im Falle einer Infektion der Durchtrittsstelle zu verhindern, dass diese vor der Entdeckung und Behandlung der Infektion das Verbindungssystem erreicht, kann ein hinreichender Abstand zwischen der Durchtrittsstelle und dem Verbindungssystem vorgesehen sein. Beispielsweise kann ein Abstand zwischen der Durchtrittsstelle und dem Verbindungssystem mindestens 5 cm, insbesondere mindestens 8 cm betragen. Damit jedoch keine unnötig große Länge der transkutanen Leitung ausgetauscht werden muss, kann der Abstand zwischen der Durchtrittsstelle und dem Verbindungssystem höchstens 20 cm, insbesondere höchstens 12 cm, betragen.

In der Regel ist das Verbindungssystem eingerichtet, eine Leistung von zumindest 2 W, insbesondere zumindest 4 W oder 5 W, drahtlos zu übertragen. Es kann vorgesehen sein, dass das Verbindungssystem eingerichtet ist, eine Leistung von maximal 30 Watt zu übertragen. Auf diese Weise eignet sich das Verbindungssystem besonders zur Energieübertragung von der Energieeinheit an die Blutpumpe. Hierbei eignet sich das vorgeschlagenen Verbindungssystem besonders, da bei dessen Verwendung nicht notwendigerweise Hautschichten zwischen den Verbindungseinheiten angeordnet sind, die bei der Übertragung der gewünschten Leistungen sonst thermisch geschädigt werden könnten.

Da das vorgeschlagene Verbindungssystem einen geringen Abstand zwischen den Verbindungseinheiten ermöglicht, kann zur zuverlässigen Energie- bzw. Datenübertragung auf geringe Baugrößen zurückgegriffen werden. Da beispielsweise lediglich vergleichsweise dünne Gehäusewände der Gehäuse der Verbindungseinheiten vonnöten sind, kann ein Abstand zwischen den gegebenenfalls vorgesehenen Spulen der Verbindungseinheiten, insbesondere während der Verwendung des Verbindungssystems, weniger als 4 mm, insbesondere weniger als 2 mm betragen. Durch den damit verbundenen hohen Wirkungsgrad des vorgeschlagenen Verbindungssystems können geringe Baugrößen erreicht werden, beispielsweise indem auf flache Komponenten zurückgegriffen wird und/oder indem Spulendurchmesser der Spulen der Verbindungseinheiten klein gewählt werden. Beispielsweise können die erste Verbindungseinheit und die zweite Verbindungseinheit eine flache Bauform aufweisen. Die erste Verbindungseinheit und die zweite Verbindungseinheit können, wenn diese berührend aufeinander liegen, eine Gesamthöhe von weniger als 20 mm, insbesondere weniger als 10 mm, aufweisen. Insbesondere durch eine geringe Höhe im Vergleich zu den Ausdehnungen in sonstigen Richtungen ist das Verbindungssystem für die Platzierung unter der Haut des Patienten, insbesondere in der Subkutis und/oder unterhalb der oberflächlichen Faszie, geeignet. Zudem kann es vorgesehen sein, dass die erste Verbindungseinheit und/oder die zweite Verbindungseinheit einen Durchmesser von höchstens 40 mm, insbesondere höchstens 30 mm, aufweisen. Insbesondere kann ein größter Gehäusedurchmesser der der ersten Verbindungseinheit und/oder der zweiten Verbindungseinheit weniger als 30 mm betragen. Der Vorteil der geringen Baugröße kommt zum Tragen, indem die Spulen bei zuverlässiger Energie- und Datenübertragung sowie geringer Gewebeschädigung einen Durchmesser aufweisen, der geringer sein kann als bei bekannten TET-Systemen. Ein Durchmesser der Spule der ersten Verbindungseinheit und/oder ein Durchmesser der Spule der zweiten Verbindungseinheit kann weniger als 40 mm, insbesondere weniger als 30 mm, beispielsweise weniger als 20 mm, betragen. In der Regel sind die Spulen derart ausgerichtet, dass diese mit einer Spulenebene entlang einer Ebene einer flächigen Ausdehnung der Verbindungseinheiten orientiert sind.

Sieht das System bei der Implantation vor, dass die zweite Verbindungseinheit, insbesondere ein induktiver Steckverbinder, durch die Haut geführt werden muss, ist es von Vorteil, wenn ein Außenquerschnitt klein ist, damit eine Inzision in die Haut klein bleibt. Somit kann alternativ zu einer flachen Ausgestaltung des Steckverbinders auch ein Rundverbinder vorgesehen sein. Wenn der Rundverbinder eine Spule zur induktiven Datenübertragung aufweist, ist diese in der Regel im Wesentlichen quer zu einer Längsrichtung der transkutanen Leitung orientiert. Eine als Rundverbinder ausgebildete zweite Verbindungseinheit, insbesondere mit vergleichsweise kleinem Außenquerschnitt von höchstens 20 mm, kann durch die Haut geführt und beispielsweise mit oben oder unten beschriebenen Methoden mit der ersten Verbindungseinheit mechanisch verbunden werden. Entsprechend kann auch die erste Verbindungseinheit ausgebildet sein. Es kann vorgesehen sein, dass auch diese durch die Haut gezogen wird und zwar in Richtung der Steuereinheit.

In einigen Ausführungen können die erste Verbindungseinheit und/oder die zweite Verbindungseinheit in ihren mit dem Körper in Verbindung zu bringenden Bestandteilen eine abgerundete Bauform aufweisen. Auf diese Weise eignen sich die erste Verbindungseinheit und die zweite Verbindungseinheit besonders für eine Verwendung innerhalb des Körpers des Patienten. Beispielsweise können Gehäuse der ersten Verbindungseinheit und/oder der zweiten Verbindungseinheit an ihrer Außenseite stufen- und/oder kantenfrei ausgebildet sein.

In einigen Ausführungen ist die erste Verbindungseinheit und/oder die zweite Verbindungseinheit verformbar ausgeführt, insbesondere elastisch verformbar und/oder flexibel. Auf diese Weise eignen sich die Verbindungseinheiten besonders für eine Implantation in den oben und unten beschriebenen Hautschichten des Patienten.

Es kann des Weiteren vorgesehen sein, dass die erste Verbindungseinheit und/oder die zweite Verbindungseinheit ein sowohl biokompatibles als auch flexibles Material, insbesondere Silikon, aufweist. Insbesondere können die gegebenenfalls vorgesehenen Spulen der ersten und/oder zweiten Verbindungseinheiten von diesem Material umgeben, insbesondere eingebettet und/oder umspritzt, sein.

Wird eine einzelne Gleichspannung oder Wechselspannung zur Blutpumpe übertragen, wird dies in der Regel mit einem Spulenpaar, d.h. einer ersten und einem zweiten Verbindungseinheit, durchgeführt. Dies erfordert eine Erzeugung der Motorphasen-Spannungen innerhalb der Blutpumpe. Sollen die Motorphasen-Spannungen, typischerweise 3 Phasen, von der externen Steuereinheit zur Blutpumpe übertragen werden, sind in der Regel auch 3 Steckverbinder notwendig. Diese können sich nach der Hautdurchtrittsstelle intrakorporal aufteilen, so dass 3 Steckverbinder einzeln platziert werden. Die drei Steckverbinder für die Motorphasen können auch in einem gemeinsamen Gehäuse untergebracht sein und so arrangiert sein, dass sie geometrisch wenig Raum einnehmen. So können beispielweise noch mehr als 3 Signale zur Blutpumpe übertragen werden.

Die Anmeldung kann sich auf eine erste Verbindungseinheit und/oder zweite Verbindungseinheit mit den oben oder unten beschriebenen Eigenschaften beziehen.

Ausführungsbeispiele werden nachfolgend anhand der Abbildungen beschrieben. Es zeigen
- Fig. 1: eine schematische Ansicht eines Herzunterstützungssystems mit einem Verbindungssystem,
- Fig. 2: eine Anordnung des Verbindungssystems unter einer Haut eines Patienten,
- Fign. 3(a) bis (c): verschiedene Ansichten des Verbindungssystems und
- Fig. 4: eine Aufsicht auf Spulen einer Verbindungseinheit des Verbindungssystems.

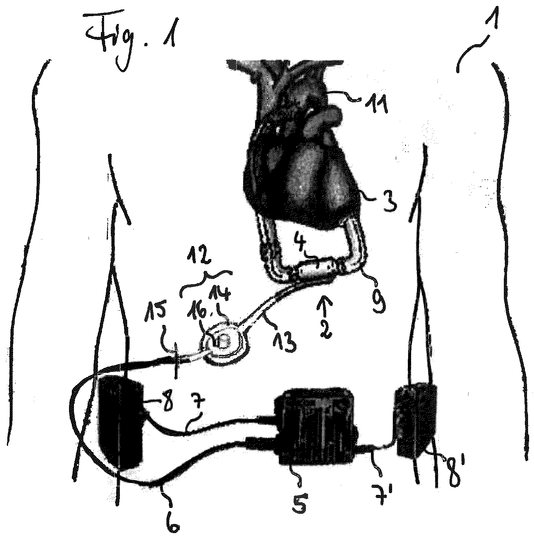

Figur 1 zeigt schematisch einen Körper 1 eines Patienten, in dem eine Blutpumpe 2 zur Unterstützung einer Funktion des Herzens 3 implantiert ist. Die Blutpumpe 2 ist als Teil eines Herzunterstützungssystems ausgebildet und weist einen typischerweise als Elektromotor mit einem Rotor ausgeführten Motor auf, der in einem biokompatiblen Pumpengehäuse 4 der Blutpumpe 2 aufgenommen ist. Das Pumpengehäuse 4 ist mit einer Steuereinheit 5 verbunden, die außerhalb des Patientenkörpers angeordnet und mit der Blutpumpe 2 über eine transkutane Leitung 6 verbunden ist. Die Steuereinheit 5 ist zudem über zwei Kabel 7, 7' mit einer als ein Paar extrakorporaler Akkus 8, 8' ausgebildeten Energieeinheit verbunden. Das Pumpengehäuse 4 ist mit einem Einlasskanal 9, über den Blut aus einer Kammer des Herzens 3 entnommen wird, und mit einer Kanüle 10, über die Blut in ein Blutgefäß 11 gefördert werden kann, verbunden.

Das Steuergerät 5 ist eingerichtet, den Motor der Blutpumpe 2 zum Fördern des Blutes anzusteuern und mit Energie zu versorgen. Zudem können beispielsweise Sensordaten von der Blutpumpe 2 an die Steuereinheit 5 übertragen werden. Hierzu ist die transkutane Leitung 6 über ein implantiertes, insbesondere vollständig innerhalb des Patientenkörpers angeordnetes, Verbindungssystem 12 mit einem vollständig implantierten Kabel 13 verbunden. Das vollständig implantierte Kabel 13 verbindet eine erste Verbindungseinheit 14 des Verbindungssystems 12 mit der Blutpumpe 2. Das transkutane Kabel 6 passiert die Haut des Patienten an einer Durchtrittsstelle 15, d.h. an einer Öffnung in der Haut des Patienten, und verbindet die Steuereinheit 15 mit einer zweiten Verbindungseinheit 16 des Verbindungssystems 12. Zur Übertragung von Daten und Energie mit einer Leistung von mehr als 2 W, insbesondere mehr als 5 W, ist die erste Verbindungseinheit 14 drahtlos mit der zweiten Verbindungseinheit 16 gekoppelt. In den gezeigten Beispielen ist eine induktive Kopplung vorgesehen, es kann aber in anderen Ausführungen auch eine kapazitive Kopplung vorgesehen sein. Stromleitende Verbindungen zwischen der ersten Verbindungseinheit und der zweiten Verbindungseinheit bestehen in der Regel nicht.

Typischerweise ist das Verbindungssystem 12 eingerichtet, elektrische Energie zur Stromversorgung der Blutpumpe 2 von der zweiten Verbindungseinheit 16 an die erste Verbindungseinheit 14 zu übertragen. Zudem ist das Verbindungssystem 12 in der Regel eingerichtet, elektrische Signale, wie beispielsweise Sensordaten oder Steuerungsdaten, sowohl von der zweiten Verbindungseinheit 16 an die erste Verbindungseinheit 14 als auch von der ersten Verbindungseinheit 14 an die zweite Verbindungseinheit 16 zu übertragen. Hierbei kann die Körperflüssigkeit des Patienten als Störgröße zu berücksichtigen sein, da sich das Verbindungssystem 12 vollständig im Körper des Patienten befindet.

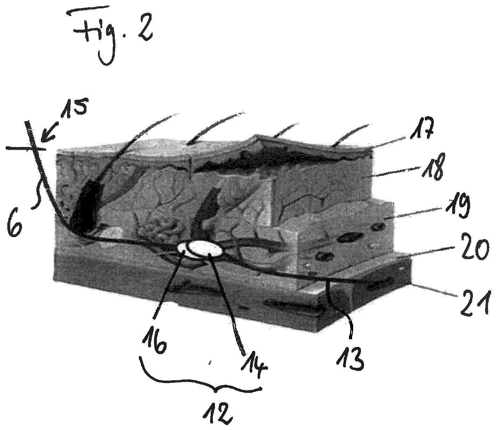

Figur 2 zeigt eine Anordnung des Verbindungssystems 12 unter der Haut des Patienten. Wiederkehrende Merkmale sind in dieser Abbildung und in den nachfolgenden Abbildungen mit den gleichen Bezugszeichen versehen. Dargestellt ist der Aufbau der Körper- und insbesondere Hautschichten umfassend die Epidermis 17, die Dermis 18, die Subkutis 19, die Faszie 20 sowie einen Muskel 21. Das Verbindungssystem 12 ist bei der Verwendung der Blutpumpe 2 bzw. des Herzunterstützungssystems in der Regel etwa 8 bis 12 cm von der Durchtrittsstelle 15 entfernt angeordnet. Das Verbindungssystem 12 mit den Verbindungseinheiten 14, 16 ist flach ausgebildet und so angeordnet, dass eine Richtung seiner flächigen Ausdehnung in einer Schichtebene der Körperschicht liegt. Das Verbindungssystem 12 kann in einer Hauttasche des Patienten implantiert sein. In dem dargestellten Beispiel ist das Verbindungssystem 12 in der Subkutis angeordnet, sodass dieses zum Austausch der zweiten Verbindungseinheit 16 und der transkutanen Leitung 6 im Falle einer Infektion der Durchtrittsstelle 15 vergleichsweise leicht zugänglich ist. Es kann aber auch vorgesehen sein, dass das Verbindungssystem 12 unterhalb der oberflächlichen Faszie angeordnet wird, sodass diese eine zusätzliche Infektionsbarriere bildet.

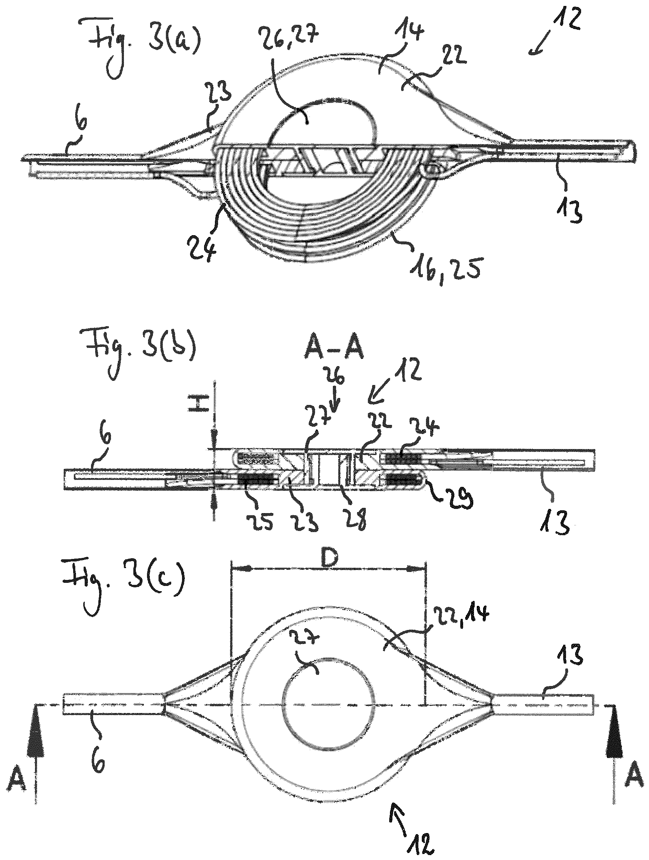

Figuren 3(a) bis (c) zeigen unterschiedliche Ansichten des Verbindungssystems 12. Die erste Verbindungseinheit 14 und die zweite Verbindungseinheit 16 weisen jeweils ein Gehäuse 22, 23 auf. Teile der Gehäuse 22, 23 sind in Fig. 3(a) nicht dargestellt, um in diesen aufgenommene Spulen 24, 25 darzustellen. Eine der Spulen 24 ist der ersten Verbindungseinheit 14 zuzuordnen, während eine Zweite der Spulen 25 der zweiten Verbindungseinheit 16 zuzuordnen ist. Die Spulen 24, 25 werden zur Energie- bzw. Datenübertragung konzentrisch übereinander angeordnet. Im gezeigten Beispiel sind die Spulen kernlos ausgeführt. Im Allgemeinen können die Spulen 24, 25, müssen aber nicht notwendigerweise, gleich groß sein. In einigen Ausführungen ist eine Ferritplatte integriert zur Erhöhung des Wirkungsgrads.

Wie die Fig. 3(b) in einer Schnittansicht zeigt, liegen die Gehäuse 22, 23 der ersten und zweiten Verbindungseinheit 14, 16 bei der Verwendung des Verbindungssystems 12 zur Energie- bzw. Datenübertragen berührend aufeinander. Die Gehäuse 22, 23 werden jeweils durch einen biokompatiblen, elastisch verformbaren Silikonkörper gebildet, mit dem die jeweilige der Spulen 24, 25 umspritzt ist. Die Gehäuse 22, 23 schließen die Spulen 24, 25 im Allgemeinen körperflüssigkeitsdicht ein und sind aus einem korrosions- und hydrolysebeständigen Material, hier Silikon, gefertigt. Die Gehäuse sind frei von scharfen Kanten und weisen eine abgerundete Außenseite auf, siehe beispielsweise das mit dem Bezugszeichen 29 gekennzeichnete Ende der zweiten Verbindungseinheit 16. Es stehen, bis auf die Leitungen 6, 13 keine Teile des Herzunterstützungssystems von den Verbindungseinheiten 14, 16 hervor.

Eine Höhe H des gesamten Verbindungssystems 12 beträgt weniger als 10 mm, wenn die Verbindungseinheiten 14, 16 berührend aufeinander liegen, sodass das Verbindungssystem 12 ein geringes Implantationsvolumen erfordert. Ein senkrechter Abstand der Spulen 24, 25 ist für eine effiziente induktive Kopplung kleiner als 2 mm, wenn die Verbindungseinheiten 14, 16 berührend aufeinander liegen.

Die Verbindungseinheiten 14, 16 sind zudem mechanisch miteinander verbunden und so zueinander ausgerichtet, dass die Spulen 24, 25, wie in den Fign. 3(a) bis (c) gezeigt, parallel und konzentrisch in einer definierten Position zueinander zu liegen kommen. Zu diesem Zweck weist das Verbindungssystem 12 ein mechanisches Verbindungsmittel 26 auf, das gleichzeitig als Ausrichtungsmittel dient. In der dargestellten Ausführung wird das Verbindungsmittel 26 durch eine Rastverbindung mit einem Paar 27, 28 ineinander greifender Rastelemente gebildet. Das Verbindungsmittel 26 ist so ausgebildet, dass es auch im Körper zuverlässig hält, wobei eine zum Lösen erforderliche Kraftwirkung mehr als 10 N, beispielsweise 20 N, betragen kann. Hinsichtlich des Verbindungsmittels kann es beispielsweise vorgesehen sein, dass die Verbindungeinheiten 14, 16 über Gewindehülsen gesichert sind (Verbindungseinheiten werden aufeinander aufgeschraubt), was insbesondere bei einem harten Gehäusematerial geeignet wäre. Alternativ kommt als Verbindungsmittel auch ein Bajonettverschluss in Betracht.

Figur 3(c) zeigt eine Aufsicht auf das Verbindungssystem 12. Ein Durchmesser D der Spulen 24, 25 beträgt beispielsweise vergleichsweise geringe 10 oder 15 mm. Ein Durchmesser der Gehäuse 22, 23 ist nur unwesentlich größer. In einigen Ausführungen weist das Verbindungssystem 12 einen Knickschutz am Übergang zwischen der Leitung 6 bzw. 13 und der Verbindungseinheit 14 bzw. 16 auf um ein Abknicken der Leitungen zu verhindern. Es kann auch ein Zugentlastungselement und/oder eine Schirmungsüberleitung zwischen der Leitung 6 bzw. 13 und der Verbindungseinheit 14 vorgesehen sein. Im dargestellten Beispiel wird der Knickschutz durch den Leitungen 6, 13 zugewandte Enden der elastischen Silikongehäuse 22, 23 gebildet.

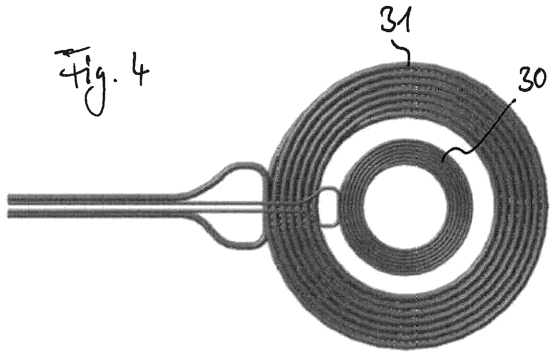

Figur 4 zeigt ein Paar konzentrisch angeordneter Spulen 30, 31 gemäß einer weiteren Ausführungsform, die wie oben beschrieben in dem Gehäuse 22 der ersten Verbindungseinheit 14 vorgesehen sein können. Hierbei bildet die Spule mit dem Bezugszeichen 31 eine Primärspule zur Energieübertragung. Die kleinere Spule mit dem Bezugszeichen 30 bildet eine Sekundärspule zur gleichzeitigen Signalübertragung. Die zweite Verbindungseinheit 16 kann entsprechend eine Primärspule und eine Sekundärspule aufweisen.

Lediglich in den Ausführungsbeispielen offenbarte Merkmale der verschiedenen Ausführungsformen können miteinander kombiniert und einzeln beansprucht werden.

## Patentansprüche

1. Verbindungssystem (12) zur Energie- und/oder Datenübertragung von und/oder zu einer implantierbaren Blutpumpe (2), umfassend eine erste Verbindungseinheit (14), die mit der Blutpumpe (2) verbindbar ist, und eine zweite Verbindungseinheit (16), die mit einer extrakorporalen Steuer- und/oder Energieeinheit (5) verbindbar ist, wobei die erste Verbindungseinheit (14) und die zweite Verbindungseinheit (16) zur drahtlosen Energie- bzw. Datenübertragung drahtlos miteinander koppelbar sind,
**dadurch gekennzeichnet, dass**
die erste Verbindungseinheit (14) und die zweite Verbindungseinheit (16) implantierbar ausgeführt sind, sodass sowohl die erste Verbindungseinheit (14) als auch die zweite Verbindungseinheit (16) zur Verwendung innerhalb eines Körpers (1) eines Patienten eingerichtet ist.

2. Verbindungssystem (12) nach Anspruch 1, **dadurch gekennzeichnet, dass** die erste Verbindungseinheit (14) zumindest eine Spule (24) aufweist und die zweite Verbindungseinheit (16) zumindest eine Spule (25) aufweist, wobei die erste Verbindungseinheit (14) und die zweite Verbindungseinheit (16) über die Spulen (24, 25) zur drahtlosen Energie- bzw. Datenübertragung induktiv miteinander koppelbar sind.

3. Verbindungssystem (12) nach einem der Ansprüche 1 oder 2, **dadurch gekennzeichnet, dass** die erste Verbindungseinheit (14) ein Gehäuse (22) aufweist und dass die zweite Verbindungseinheit (16) ein Gehäuse (23) aufweist, wobei das Gehäuse (22) der ersten Verbindungseinheit (14) und das Gehäuse (23) der zweiten Verbindungseinheit (16) flüssigkeitsdicht, insbesondere dicht für Körperflüssigkeiten, ausgeführt sind.

4. Verbindungssystem (12) nach einem der Ansprüche 1 bis 3, **gekennzeichnet durch** ein mechanisches Verbindungsmittel (26), durch das die erste Verbindungseinheit (14) und die zweite Verbindungseinheit (16) lösbar miteinander verbindbar sind.

5. Verbindungssystem (12) nach Anspruch 4, **dadurch gekennzeichnet, dass** das mechanische Verbindungsmittel (26) eine Verbindung zwischen der ersten Verbindungseinheit (14) und der zweiten Verbindungseinheit (16) vermittelt, deren Lösen eine Kraftwirkung von zumindest 10 N, insbesondere zumindest 15 N, erfordert.

6. Verbindungssystem (12) nach Anspruch 4 oder 5, **dadurch gekennzeichnet, dass** das mechanische Verbindungsmittel (26) ein Schraubverschluss, ein Bajonettverschluss, ein Rastverschluss oder und/oder ein Schnappverschluss ist.

7. Verbindungssystem (12) nach einem der Ansprüche 1 bis 6, **gekennzeichnet durch** ein mechanisches Ausrichtungsmittel (26), durch das die erste Verbindungseinheit (14) und die zweite Verbindungseinheit (16) zueinander ausrichtbar sind, sodass die erste Verbindungseinheit (14) und die zweite Verbindungseinheit (16) in einer vordefinierten Ausrichtung zueinander zu liegen kommen.

8. Verbindungssystem (12) nach einem der Ansprüche 1 bis 7, **dadurch gekennzeichnet, dass** dieses eingerichtet ist, eine Leistung von zumindest 2 W, insbesondere zumindest 4 W, drahtlos zu übertragen.

9. Verbindungssystem (12) nach einem der Ansprüche 1 bis 8, **dadurch gekennzeichnet, dass** die erste Verbindungseinheit (14) und die zweite Verbindungseinheit (16) eine flache Bauform aufweisen.

10. Verbindungssystem (12) nach einem der Ansprüche 1 bis 9, **dadurch gekennzeichnet, dass** die erste Verbindungseinheit (14) und die zweite Verbindungseinheit (16), wenn diese berührend aufeinander liegen, eine Gesamthöhe von weniger als 20 mm, insbesondere weniger als 10 mm, aufweisen.

11. Verbindungssystem (12) nach einem der Ansprüche 1 bis 10, **dadurch gekennzeichnet, dass** die erste Verbindungseinheit (14) und die zweite Verbindungseinheit (16) einen Durchmesser von höchstens 40 mm, insbesondere höchstens 30 mm, aufweisen.

12. Verbindungssystem (12) nach einem der Ansprüche 1 bis 11, **dadurch gekennzeichnet, dass** die erste Verbindungseinheit (14) und die zweite Verbindungseinheit (16) in ihren mit dem Körper (1) in Verbindung zu bringenden Bestandteilen eine abgerundete Bauform aufweisen.

13. Verbindungssystem (12) nach einem der Ansprüche 1 bis 12, **dadurch gekennzeichnet, dass** die erste Verbindungseinheit (14) und/oder die zweite Verbindungseinheit (16) verformbar ausgeführt ist.

14. Verbindungssystem (12) nach einem der Ansprüche 1 bis 13, **dadurch gekennzeichnet, dass** die erste Verbindungseinheit (14) und/oder die zweite Verbindungseinheit (16) Silikon aufweist.

15. Herzunterstützungssystem, umfassend ein Verbindungssystem (12) nach einem der vorhergehenden Ansprüche und weiterhin umfassend die Blutpumpe (2) und/oder die extrakorporale Steuer- und/oder Energieeinheit (5).
